Europäisches Patentamt

European Patent Office

Office européen des brevets

(11) Veröffentlichungsnummer: **0 037 012**
**A2**

# (12) EUROPÄISCHE PATENTANMELDUNG

(21) Anmeldenummer: **81102018.9**

(22) Anmeldetag: **18.03.81**

(51) Int. Cl.³: **A 61 B 10/00**

(30) Priorität: **28.03.80 DE 3012173**

(43) Veröffentlichungstag der Anmeldung:
**07.10.81 Patentblatt 81/40**

(84) Benannte Vertragsstaaten:
**AT CH DE FR GB LI NL**

(71) Anmelder: **SIEMENS AKTIENGESELLSCHAFT Berlin und München**
**Postfach 22 02 61**
**D-8000 München 22(DE)**

(72) Erfinder: **Haerten, Rainer, Dr.**
**Rt.2 Box 386 Shenandoah**
**Long Grove Illinois 60047(US)**

(54) **Ultraschall-Bildgerät.**

(57) Ultraschall-Bildgerät mit Ultraschall-Applikator zur zeilenweisen Ultraschall-Abtastung und mit einem Gerät zur Aufzeichnung eines Echosichtbildes. Ziel der Erfindung ist es, ein Bildgerät aufzubauen, das bei beliebiger Lage des Applikationsortes keine Ankoppelprobleme aufwirft; ferner soll auch insbesondere die Anwendung im Brustbereich zur Gewinnung subcostaler Schnittbilder möglich sein. Dieses Ziel wird erfindungsgemäß dadurch erreicht, daß ein bereits zeilenweise abtastender Applikator (1) zum Zwecke der Schwenkung oder Drehung der Abtastfläche (6,7) auch zusätzlich noch um eine solche Schwenk-oder auch Drehachse (3) in vorgebbarem Winkelbereich, z.B. durch Motorantrieb (2), schwenkbar oder drehbar gehaltert ist, die bei Applikation dem jeweiligen Applikationsort fest zugeordnet ist.

FIG 1

SIEMENS AKTIENGESELLSCHAFT          Unser Zeichen
Berlin und München                  VPA 80 P 5043   E

Ultraschall-Bildgerät

Die Erfindung bezieht sich auf ein Ultraschall-Bildgerät mit Ultraschall-Applikator zur zeilenweisen Ultraschall-Abtastung und mit einem Gerät zur Aufzeichnung eines Echosichtbildes.

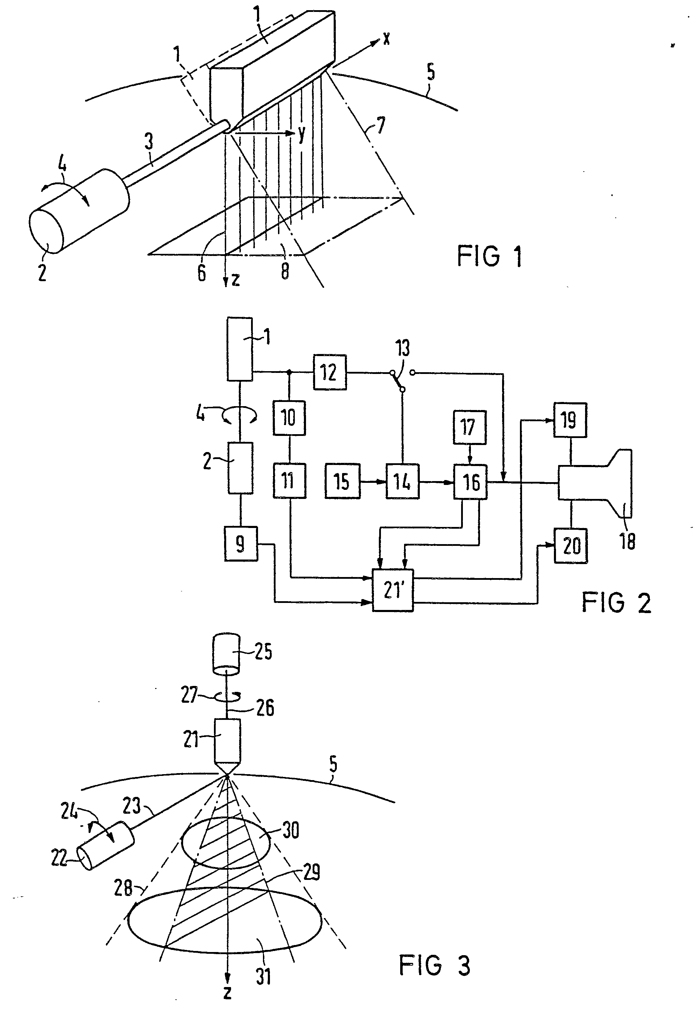

Ein Ultraschall-Bildgerät dieser Art ist z.B. durch den Aufsatz "A New Real-Time Ultrasonic Diagnostic System for Dynamic and Still Images" von K. Ito et al aus der Zeitschrift Medical Electronics, JEE, December '78, Seiten 60 bis 64 bekannt. Der Ultraschall-Applikator dieses Gerätes ist speziell ein Ultraschall-Array, das durch elektronische Strahlfortschaltung bereits zeilenweise ein Untersuchungsgebiet abtastet. Gemäß der Figur 2 des Aufsatzes kann dieses Ultraschall-Array durch Motorantrieb auf der Oberfläche des Untersuchungsobjektes parallel zu sich selbst verschoben werden. Bei reinem B-Mode erhält man so in zeitlicher Aufeinanderfolge zueinander parallele Schnittbilder des Untersuchungsobjektes. Bei Einsatz geeigneter Zeittore ist auch eine Abtastung im C-Mode oder im F-Mode möglich. Im C-Mode werden ebene Schnitt erhalten, die senkrecht zur Strahlrichtung in vorgebbarer Tiefe des Untersuchungsobjektes verlaufen. Der F-Mode liefert bei entsprechender Programmierung der Zeitverzögerung des Zeitgliedes beliebig geformte Flächen.

Die spezielle Art der Verschiebung des Ultraschall-Arrays über eine große Fläche bringt Probleme hinsichtlich korrekter Ankopplung zwischen Array und Körperoberfläche. Die Anwendung ist außerdem auf Weichgewebe,

Kue 5 Kof / 17.03.1980

z.B. Bauchregion, beschränkt. Subcostale Schichtbilder
können wegen der Abschatteffekte der Rippen nicht erhalten werden.

Aufgabe der Erfindung ist es, ein Ultraschall-Bildgerät
der eingangs genannten Art aufzubauen, das bei beliebi-.
ger Lage des Applikationsortes keine Ankoppelprobleme
aufwirft; ferner soll auch insbesondere die Anwendung
im Brustbereich zur Gewinnung subcostaler Schnittbilder, z.B. des Herzens oder auch der Leber, möglich sein.

Die Aufgabe wird erfindungsgemäß dadurch gelöst, daß
ein bereits zeilenweise abtastender Applikator zum
Zwecke der Schwenkung oder Drehung der Abtastfläche
auch zusätzlich noch um eine solche Schwenk- oder auch
Drehachse in vorgebbarem Winkelbereich, z.B. durch
programmierbaren Motorantrieb, schwenkbar oder drehbar gehaltert ist, die bei Applikation dem jeweiligen
Applikationsort fest zugeordnet ist.

Die Erfindung erlaubt durch Ebenenschwenkung und/oder
Drehung großflächige Abtastung bei kleiner ortsfester
Ankoppelfläche; die Ankopplung ist also einfach und
wirft keine Probleme auf.  Eine Anwendung im Brustbereich zur Gewinnung subcostaler Schichtbilder ist
wegen der kleinen Ankoppelfläche, die einen problemlosen Ansatz zwischen oder unter den Rippen gewährleistet, jederzeit möglich. Der Einsatz ist nicht auf die
Gewinnung von Bildern im B-Mode beschränkt; durch Hinzufügung einer geeigneten Zeittorschaltung kann ebenso
wie beim Bildgerät des Aufsatzes aus Medical Electronics
auf Betrieb im C-Mode oder F-Mode umgeschaltet werden,
ohne jedoch die Nachteile dieses Gerätes in Kauf nehmen zu müssen. Desgleichen ist bei Bedarf auch ein
Übergang auf zweidimensionale Doppler-Bild-Darstellung möglich.

Weitere Vorteile und Einzelheiten der Erfindung ergeben sich aus der nachfolgenden Beschreibung zweier Ausführungsbeispiele anhand der Zeichnung in Verbindung mit den Unteransprüchen.

Es zeigen:

Fig. 1 ein erstes Ausführungsbeispiel mit schwenkbarem Ultraschall-Array als Ultraschall-Applikator,

Fig. 2 das Prinzipschaltbild eines Bildgerätes, das ein solches schwenkbares Ultraschall-Array als Applikator verwendet,

Fig. 3 ein Ausführungsbeispiel für einen Sektorabtastkopf, der zusätzlich zur mechanischen Sektorschwenkbewegung auch noch gedreht wird.

In der Fig. 1 ist ein Ultraschall-Array zur zeilenweisen Ultraschall-Abtastung mit 1 bezeichnet. Das Ultraschall-Array ist durch Motorantrieb 2 um eine Drehachse 3 in Richtung des Schwenkpfeiles 4 schwenkbar gehaltert. Die Drehachse 3 zeigt in Längsrichtung der Applikationsfläche des Arrays 1. Der Anlenkpunkt liegt an einer Seitenfläche nahe der Applikationsfläche des Arrays. Bei Auflage auf der Oberfläche eines Untersuchungsobjektes, z.B. zwischen den Rippen eines zu untersuchenden Patienten, sind aufgrund der Schwenkbewegung des Ultraschall-Arrays 1 ähnlich wie bei einem Sektorabtaster für den Einzelstrahl Verschwenkungen der Abtastebene möglich. In der Fig. 1 ist beispielsweise die mittlere Abtastebene in Mittenstellung des Applikators mit 6 angedeutet, während sich bei der nur gestrichelt angedeuteten linken Schräglage des Arrays die geschwenkte Abtastebene 7 ergibt. Bei Übergang auf C- oder F-Mode erhält man bei Einsatz geeigneter Zeittorschaltungen beispielsweise die Schnittebene 8.

Das Prinzipschaltbild des Bildgerätes, das z.B. in Verbindung mit einem Ultraschall-Array gemäß Figur 1 arbeitet, ist in der Figur 2 dargestellt. Hier ist dem
Schwenkmotor 4 ein Winkelgeber 9 zugeordnet, der für
jede Schwenkbewegung die für den Bildaufbau benötigten
Winkelpositionssignale liefert. Der Sender für die Sendeimpulse ist mit 10 bezeichnet. Er wird von einem
zentralen Taktgeber 11 gesteuert. Die empfangenen
Echosignale werden von einem Empfangsverstärker 12 aufgenommen und über einen Wahlschalter 13 entweder direkt
(zur Real-time-Darstellung) oder über eine Zwischenverarbeitungsschaltung 14 bis 17 einer Elektronenstrahlröhre 18 mit Zeilenkippgenerator 19 und Bildkippgenerator 20 sowie Adressrechner 21' zur Aufzeichnung zum
Echoschnittbild zugeleitet. Die direkte Zuleitung (oder
auch gegebenenfalls Zuleitung über Zwischenspeicher)
führt zur Darstellung im B-Mode. Darstellung im C-Mode
oder gegebenenfalls auch F-Mode ist in der gezeigten
Schaltstellung des Wahlschalters 13 möglich. In dieser
Schaltstellung werden nämlich die Echosignale des Verstärkers 12 auf ein Zeittor 14 gegeben, das gesteuert
von einem Tiefenwertgeber 15 nur Echosignale aus einer
vorgebbaren Tiefe des Untersuchungsobjektes hindurchläßt. Die so hindurchgelassenen tiefenselektierten Echosignale werden in einem Bildspeicher 16 mit beigeordnetem Rechner 17 für Bildverarbeitung, insbesondere ein-
oder mehrdimensionale ortsfrequenzabhängige Filterung,
gespeichert und von dort zur Aufzeichnung am Bildschirm
der Bildröhre 18 abgerufen. Der Tiefenwertgeber 15 ist
vorzugsweise ein Programmgeber, der konstante oder nach
beliebigen Funktionen variable Tiefenwerte liefert. Der
Rechner 14 gewährleistet, daß im Falle des Betriebs im
C-Mode oder auch F-Mode eine gleichmäßige Auflösung über
den Gesamtflächenbereich gegeben ist.

Die Figur 3 zeigt eine Ausführungsform mit einem Sektorabtastkopf 21. Dieser Sektorabtastkopf wird mechanisch in der üblichen Weise durch Schwenkmotor 22 um eine Schwenkachse 23 in Richtung des Doppelpfeiles 24 geschwenkt. Der Schwenkbewegung im vorgebbaren Winkelbereich wird nun zusätzlich durch Drehmotor 25 eine Drehbewegung des Abtastkopfes 21 um die zentrale Drehachse 26 in Richtung des Drehpfeiles 27 überlagert. Der Abtastsektor wird also gedreht und es ergibt sich der in der Figur 3 angedeutete Abtastkegel 28. Die Darstellung kann wieder im B-Mode erfolgen, d.h. es wird am Bildschirm der Oszillographenröhre der jeweilige in Schritten gedrehte Sektor 29 abgebildet. Ebensogut können durch Umschaltung auf C-Mode Schnittebenen nach Art der Ebenen 30 und 31 bzw. durch Umschaltung auf F-Mode auch anders gestaltete, z.B. gekrümmte, Flächen od.dgl. gewonnen werden. Das Prinzipschaltbild für ein Ultraschall-Bildgerät, das mit einem Applikator gemäß Figur 3 arbeitet, entspricht in den wesentlichen Grundzügen jenem der Figur 2. Zusätzlich zu den Winkelsignalen der Sektorbewegung müssen dann lediglich auch noch die Winkelsignale der Drehbewegung erfaßt und bei der Bilddarstellung berücksichtigt werden.

3 Figuren

9 Patentansprüche

Patentansprüche

1. Ultraschall-Bildgerät mit Ultraschall-Applikator zur zeilenweisen Ultraschall-Abtastung und mit einem Gerät zur Aufzeichnung eines Echosichtbildes, d a d u r c h   g e k e n n z e i c h n e t ,   daß ein bereits zeilenweise abtastender Applikator (1 oder 21) zum Zwecke der Schwenkung oder Drehung der Abtastfläche (6, 7 oder 29) auch zusätzlich noch um eine solche Schwenk- oder auch Drehachse (3 oder 26) in vorgebbarem Winkelbereich, z.B. durch programmierbaren $M_o$torantrieb (2 oder 25), schwenkbar oder drehbar gehaltert ist, die bei Applikation dem jeweiligen Applikationsort fest zugeordnet ist.

2. Ultraschall-Bildgerät nach Anspruch 1,   d a - d u r c h   g e k e n n z e i c h n e t ,   daß ein durch elektronische Strahlfortschaltung bereits zeilenweise abtastendes Ultraschall-Array (1) durch Motorantrieb (2) um eine Schwenkachse (3) schwenkbar gehaltert ist, die im Bereich der Applikationsfläche in Längsrichtung des Arrays (1) verläuft.

3. Ultraschall-Bildgerät nach Anspruch 2,   d a - d u r c h   g e k e n n z e i c h n e t ,   daß der Schwenkmotor (2) mit seiner Schwenkachse (3) au wenigstens einer Seitenfläche des Ultraschall-Arrays nahe der Applikationsfläche angreift.

4. Ultraschall-Bildgerät nach Anspruch 1,   d a - d u r c h   g e k e n n z e i c h n e t ,   daß ein Abtastkopf (21) für Sektorabtastung, der zur Strahlschwenkung entweder selbst mechanisch, z.B. durch $M_o$torantrieb (22), geschwenkt wird oder dessen Ultraschallstrahl ohne mechanische Sektorschwenkbewegung lediglich aufgrund elektronischer Steuerung geschwenkt wird,um eine unter vorgebbarem Winkel, z.B. senkrecht, zur Applikationsfläche verlaufende Achse (26), z.B. durch einen Dreh- oder Schwenkmotor (25), dreh- oder schwenkbar gehaltert ist.

5. Ultraschall-Bildgerät nach einem der Ansprüche 1 bis 4, d a d u r c h   g e k e n n z e i c h n e t , daß die Abtastung im B-Mode oder durch Hinzufügung einer geeigneten Zeittorschaltung (14, 15) auch im C-Mode oder F-Mode erfolgt.

6. Ultraschall-Bildgerät nach Anspruch 5,   d a - d u r c h   g e k e n n z e i c h n e t ,   daß die Echosignale aus den unterschiedlichen Abtastschichten dem Aufzeichnungsgerät (Elektronenstrahlröhre 18) direkt oder über Bildspeicher (16) zur Aufzeichnung zuleitbar sind.

7. Ultraschall-Bildgerät nach Anspruch 6,   d a - d u r c h   g e k e n n z e i c h n e t ,   daß für C-Mode oder F-Mode dem Bildspeicher (16) ein Rechner (17) zur Bildverarbeitung, insbesondere zur ein- oder mehrdimensionalen, ortsfrequenzabhängigen Filterung, der Echosignale beigeordnet ist.

8. Ultraschall-Bildgerät nach einem der Ansprüche 1 bis 7, d a d u r c h   g e k e n n z e i c h n e t , daß für C- oder F-Mode am Dreh- oder Schwenkmotor die Winkelschritte so vorprogrammiert sind, daß sich unabhängig von der gewählten C- oder F-Schichttiefe ein äquidistantes, zweidimensionales Rastermaß für die Bildpunkte ergibt.

9. Ultraschall-Bildgerät nach einem der Ansprüche 1 bis 8, d a d u r c h   g e k e n n z e i c h n e t , daß zum eventuellen Übergang auf zweidimensionale Ultraschall-Doppler-Bilddarstellung in die Empfangsschaltung ein Dopplergerät zur Erfassung von Dopplersignalen aus bewegten Teilen des Untersuchungsobjektes einschaltbar ist.

FIG 1

FIG 2

FIG 3